# EUROPÄISCHE PATENTANMELDUNG

(11) **EP 1 247 502 A1**
(43) Veröffentlichungstag der Anmeldung: **09.10.2002**
(21) Anmeldenummer: 02006444.0
(22) Anmeldetag: 22.03.2002
(51) Int. Cl.: A61F 2/34

(54) **Beckenpfanne eines künstlichen Hüftgelenkes**

(30) Priorität: 04.04.2001 DE 20105862 U
(71) Anmelder: ESKA Implants GmbH & Co., 23556 Lübeck (DE)
(72) Erfinder: Grundei, Hans, Dr., 23558 Lübeck (DE)
(74) Vertreter: Fuchs Mehler Weiss & Fritzsche

(57) **Zusammenfassung**

Es wird eine Beckenpfanne (1) eines künstlichen Hüftgelenkes zum Einsatz in einem Acetabulum beschrieben, deren Außenseite wenigstens abschnittsweise eine dreidimensionale, offenmaschige Raumnetzstruktur (2) aufweist. Sie weist ein zusätzliches Fixationselement (3) für die Sekundärfixation im Acetabulum auf, das von der Außenseite der Beckenpfanne (1) abragt.

Das zusätzliche Fixationselement (3) ist als vom Grundkörper (4) polwärts abragende Anschlagslasche ausgebildet, die vom Grundkörper (4) zunächst in einem Winkel α im Bereich von 30° bis 50° zur Beckeneingangsebene (B) abragt und im weiteren Verlauf in sich verdreht ist. Sie ist dann zu der senkrecht auf der Beckeneingangsebene (B) stehenden Polachse (P) in einem Winkel (β) im Bereich von 10° bis 30° torquiert.

## Beschreibung

Die vorliegende Erfindung betrifft eine Beckenpfanne für ein künstliches Hüftgelenk zum Einsatz in einem Acetabulum, deren Außenseite wenigstens abschnittsweise eine dreidimensionale, offenmaschige Raumnetzstruktur aufweist, mit einem zusätzlichen Fixationselement für die Sekundärfixation, das von ihrer Außenseite abragt.

Eine solche Beckenpfanne ergibt sich beispielsweise aus der DE 39 18 970 C. Die richtige Einbaulage der Beckenpfanne im Acetabulum mit dem patientenindividuell einzustellenden Beckeneingangswinkel und Antekurvationswinkel stellt insbesondere für unerfahrene Operateure eine Schwierigkeit dar. Im Zusammenspiel mit der natürlichen Torquiertheit des Femurknochens führen falsche Winkel dazu, daß der Bewegungsablauf nach der Implantation eines künstlichen Hüftgelenkes nicht mehr physiologisch nachgebildet ist. Oftmals neigen Operateure dazu, durch ein zu festes Eindrücken der Beckenpfanne in das Acetabulum einen zu kleinen Antekurvationswinkel und einen zu großen Beckeneingangswinkel zu erzeugen.

Vor diesem Hintergrund ist es nun die Aufgabe der vorliegenden Erfindung, eine Beckenpfanne der eingangs erwähnten Art so weiter zu bilden, daß eine Hilfe für die korrekte Einstellung des Beckeneingangswinkels und des Antekurvationswinkels bereitgestellt wird.

Gelöst wird diese Aufgabe durch eine Beckenpfanne mit den Merkmalen des kennzeichnenden Teiles des Anspruches 1. Vorteilhafte Weiterbildungen ergeben sich aus den Unteransprüchen.

Demgemäß wird vorgeschlagen, daß das zusätzliche Fixationselement als vom Grundkörper polwärts abragende Anschlagslasche ausgebildet ist. Diese erstreckt sich vom Grundkörper der Beckenpfanne zunächst in einem Winkel im Bereich von 30° bis 50° zur Beckeneingangsebene und verdreht sich dann im weiteren Verlauf, so daß sie zu der senkrecht auf der Beckeneingangsebene stehenden Polachse in einem Winkel von 10° bis 30° torquiert ist. Diese flügelartig ausgebildete Anschlagslasche ist dazu vorgesehen, sich beim Einpressen der Beckenpfanne in das Acetabulum an die Darmbeinschaufel anzulegen und so ein weiteres Einkippen der Beckenpfanne in das Acetabulum zu vermeiden. Es hat sich bei der Untersuchung einer ganzen Reihe von Patienten herausgestellt, daß die Winkelverhältnisse bei vielen Patienten gleich sind. Daher ist es möglich, die erfindungsgemäße Beckenpfanne in größeren Stückzahlen in den Kliniken zu bevorraten.

Die Anschlagslasche ermöglicht es auch etwas unerfahrenen Operateuren, die Pfanne mit dem richtigen Beckeneingangswinkel und Antekurvationswinkel zu implantieren. Gleichzeitig kann sie auch wie ein Stopper wirken, wenn nämlich das Acetabulum weitgehend zerstört ist, so daß die Beckenpfanne keinen ausreichenden Halt im Acetabulum findet.

Insbesondere für den vorerwähnten Fall ist eine vorteilhafte Weiterbildung der Beckenpfanne vorgesehen, die nämlich mit einem in Abweichung von einer idealisierten, kalottenförmigen Pfanne nach einer Seite hin ausladenden Bereich ausgebildet ist, derart, daß sie in Aufsicht gesehen eine im wesentlichen eiförmige und in Seitenansicht gesehen eine halbeiförmige Kontur aufweist. Die derartig ausgestaltete Beckenpfanne füllt größere Bereiche im Restacetabulum aus als dies mit einer kalottenförmigen Beckenpfanne der Fall wäre.

Zur Sekundärfixation ist gemäß einer vorteilhaften Weiterbildung vorgesehen, daß auch die dem Beckenknochen zuzuwendende Seite der Anschlagslasche mit einer dreidimensionalen, offenmaschigen Raumnetzstruktur versehen ist. Auch in diese Raumnetzstruktur kann dann Knochenmaterial einwachsen und für die dauerhafte Fixation der Beckenpfanne sorgen.

Gemäß einer noch vorteilhafteren Weiterbildung ist vorgesehen, daß die Anschlagslasche um eine Strecke von bis zu 10 mm von der Basiskante der Pfanne in Richtung auf den Pol versetzt ist. Bildlich gesprochen wandert die flügelartige Anschlagslasche Richtung Pol. Hierdurch kann je nach Indikation ein anderes Bild des Implantates im Acetabulum erzeugt werden.

Die Erfindung wird anhand eines Ausführungsbeispieles näher erläutert. Hierbei zeigt:
- Fig. 1: die Beckenpfanne im seitlichen Schnitt und
- Fig. 2: die Beckenpfanne in Aufsicht auf den Polbereich.

Gleiche Teile sind nachfolgend mit denselben Bezugszeichen versehen.

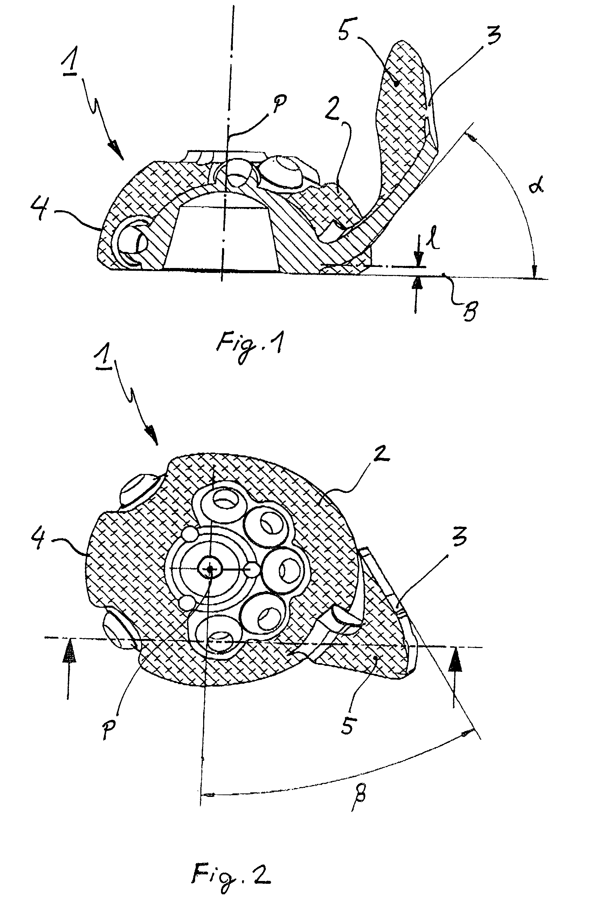

Aus Figur 1 ist ersichtlich, daß die Beckenpfanne 1 mit ihrer dreidimensionalen, offenmaschigen Raumnetzstruktur 2 mit einer flügelartigen Anschlagslasche 3 versehen ist. Diese erstreckt sich vom Grundkörper 4 der Beckenpfanne 1 polwärts. Zunächst verläuft die Anschlagslasche 3 von der Basiskante der Beckenpfanne 1 mit der Beckeneingangsebene B mit einem Versatz 1 in einem Winkel α, der zwischen 30° bis 50° beträgt, zur Beckeneingangsebene B. Etwa auf der halben Länge der Anschlagslasche 3 ist diese torquiert, und zwar so, daß sie zu der Polachse P, die senkrecht auf der Beckeneingangsebene B steht, einen Winkel β (Figur 2) im Bereich von 10° bis 30° einschließt.

Vorliegend ist auch die dem Darmbein zuzuwendende Seite 5 der Anschlagslasche 3 mit der offenmaschigen, dreidimensionalen Raumnetzstruktur versehen, damit sich auch darin Knochenmaterial einorganisieren kann.

Besonders in der Aufsicht gemäß Figur 2 ist erkennbar, daß die Beckenpfanne 1 nicht kalottenförmig ausgebildet ist, sondern zu einer Seite hin, in Figur 2 nach rechts hin, einen ausladenden Bereich aufweist, so daß die Aufsicht eine eiförmige Kontur darstellt. In Figur 1 äußert sich diese Ausbildung durch die halbeiförmige Kontur der Seitenansicht. Diese Ausbildung erlaubt es, größere Bereiche in dem Restacetabulum auszufüllen als dies mit einer rein kalottenförmig ausgebildeten Pfanne möglich wäre.

## Patentansprüche

1. Beckenpfanne (1) eines künstlichen Hüftgelenkes zum Einsatz in einem Acteabulum, deren Außenseite wenigstens abschnittsweise eine dreidimensionale, offenmaschige Raumnetzstruktur (2) aufweist, mit einem zusätzlichen Fixationselement (3) für die Sekundärfixation, das von ihrer Außenseite abragt,
dadurch gekennzeichent,
daß das zusätzliche Fixationselement (3) als vom Grundkörper (4) polwärts abragende Anschlagslasche ausgebildet ist, die vom Grundkörper (4) zunächst in einem Winkel α im Bereich von 30° bis 50° zur Beckeneingangsebene (B) abragt und im weiteren Verlauf in sich verdreht ist, so daß sie zu der senkrecht auf der Beckeneingangsebene stehenden Polachse (P) in einem Winkel β im Bereich von 10° bis 30° torquiert ist.

2. Beckenpfanne (1) nach Anspruch 1, bei der auch die dem Beckenknochen zuzuwendende Seite (5) der Anschlagslasche (3) mit einer dreidimensionalen, offenmaschigen Raumnetzstruktur versehen ist.

3. Beckenpfanne (1) nach Anspruch 1 oder 2, mit einem in Abweichung von einer idealisierten kalottenförmigen Pfanne nach einer Seite hin ausladenden Bereich, derart, daß sie in Aufsicht gesehen, eine im wesentlichen eiförmige und in Seitenansicht gesehen eine halbeiförmige Kontur aufweist.

4. Beckenpfanne (1) nach einem der Ansprüche 1 bis 3, bei der die Anschlagslasche (3) um eine Strecke 1 von der Basiskante der Pfanne (1) versetzt ist, wobei Null kleiner 1 kleiner 1 cm.
